# EUROPEAN PATENT APPLICATION

(11) **EP 4 276 840 A1**
(43) Date of publication of application: **15.11.2023**
(21) Application number: 21917616.1
(22) Date of filing: 07.12.2021
(51) Int. Cl.: G16C 20/50, G06F 16/28, G06N 5/04, G06N 99/00

(54) **INFORMATION PROCESSING DEVICE, INFORMATION PROCESSING METHOD, AND INFORMATION PROCESSING PROGRAM**

(30) Priority: 07.01.2021 JP 2021001611
(71) Applicant: FUJIFILM Corporation, Tokyo 106-8620 (JP)
(72) Inventor: YARIMIZU, Hirokazu, Tokyo 106-8620 (JP); HIKIDA, Yasushi, Tokyo 106-8620 (JP)
(74) Representative: Meissner Bolte Partnerschaft mbB
(86) International application number: PCT/JP2021/044993
(87) International publication number: WO 2022/149395

(57) **Abstract**

An information processing apparatus includes at least one processor. The processor receives input of structure data indicating a structure of a chemical substance and an evaluation function for evaluating specific performance of the chemical substance; extracts a known chemical substance having the same basic structure as a basic structure of an input structure indicated by the input structure data from a database in which structure data indicating a structure of a chemical substance is recorded for each of a plurality of known chemical substances; generates a novel structure in which the input structure is modified based on the structure of the extracted known chemical substance or a novel structure in which the structure of the extracted known chemical substance is modified; derives an index value related to the specific performance for the generated novel structure; derives an evaluation value of the novel structure based on the derived index value and the evaluation function; and displays the novel structure according to the evaluation value.

## Description

### BACKGROUND OF THE INVENTION

### 1. Field of the Invention

The disclosed technology relates to an information processing apparatus, an information processing method, and an information processing program.

### 2. Description of the Related Art

The following technologies are known as technologies related to design support for chemical substances. For example, JP2006-323833A discloses a method for designing a physiologically active compound, comprising: (1) a first step of preparing a geometry of a physiologically active compound to be designed by extracting atomic coordinates from a compound having a specific physiological activity and a known structure, (2) a second step of acquiring a molecular structure of a candidate compound by arranging possible combinations of atomic species so as to satisfy a relationship of bond order between atoms with respect to the geometry prepared in the first step, and (3) a third step of evaluating the molecular structure of the candidate compound acquired in the second step by an activity score obtained from a model predicting the physiological activity of the compound.

JP2001-058962A discloses a molecular structure development support system comprising: an input device that inputs target required properties and library creation conditions; a molecular structure extraction device including a molecular structure library creation unit that creates a molecular structure library by comprehensively storing molecular structures that can be theoretically generated based on the library creation conditions, and a property evaluation unit that extracts a molecular structure expected to have properties that match the required properties by evaluating the properties of the molecular structures stored in the molecular structure library using a computational scientific method; and an output device that outputs the molecular structure extracted by the molecular structure extraction device.

### SUMMARY OF THE INVENTION

In a structural design of a chemical substance, a designer designs a structure of the chemical substance through an editor. In a case where the structure of the chemical substance is input, the editor outputs a molecular weight and a performance index value according to the input structure. This information is very important for the structural design of the chemical substance for the purpose of producing a chemical substance exhibiting desired performance. Therefore, the designer always keeps in mind the performance index value and the like output from the editor at the time of designing. An existing editor can output the molecular weight and the performance index value according to the input structure, but does not have a function of presenting a structure of a chemical substance exhibiting desired performance. Therefore, the structural design of the chemical substance exhibiting the desired performance is performed by trial and error of the designer, which imposes a heavy burden on the designer.

The disclosed technology has been made in view of the above points, and an object of the disclosed technology is to support a structural design of a chemical substance exhibiting desired performance.

An information processing apparatus according to the disclosed technology comprises at least one processor, in which the processor receives input of structure data indicating a structure of a chemical substance and an evaluation function for evaluating specific performance of the chemical substance; extracts a known chemical substance having the same basic structure as a basic structure of an input structure indicated by the input structure data from a database in which structure data indicating a structure of a chemical substance is recorded for each of a plurality of known chemical substances; generates a novel structure in which the input structure is modified based on the structure of the extracted known chemical substance or a novel structure in which the structure of the extracted known chemical substance is modified; derives an index value related to the specific performance for the generated novel structure; derives an evaluation value of the novel structure based on the derived index value and the evaluation function; and displays the novel structure according to the evaluation value.

The processor may generate the novel structure by adding a partial structure associated with the basic structure of the extracted known chemical substance to the input structure. The processor may generate the novel structure by deleting a partial structure associated with the basic structure of the input structure from the input structure.

The processor may display a difference between the novel structure and the input structure in a recognizable manner. The processor may rank a plurality of the novel structures based on the evaluation value, and display the plurality of the novel structures in a manner in which a result of the ranking is recognizable. The processor may perform derive an index value related to the specific performance for the input structure, and display the index values derived for each of the input structure and the novel structure. The processor may display only a novel structure of which the evaluation value is equal to or greater than a threshold value among a plurality of the generated novel structures.

An information processing method according to the disclosed technology is a method in which a processor of an information processing apparatus executes a process comprising: receiving input of structure data indicating a structure of a chemical substance and an evaluation function for evaluating specific performance of the chemical substance; extracting a known chemical substance having the same basic structure as a basic structure of an input structure indicated by the input structure data from a database in which structure data indicating a structure of a chemical substance is recorded for each of a plurality of known chemical substances; generating a novel structure in which the input structure is modified based on the structure of the extracted known chemical substance or a novel structure in which the structure of the extracted known chemical substance is modified; deriving an index value related to the specific performance for the generated novel structure; deriving an evaluation value of the novel structure based on the derived index value and the evaluation function; and displaying the novel structure according to the evaluation value.

An information processing program according to the disclosed technology is a program for causing a processor of an information processing apparatus to execute a process comprising: receiving input of structure data indicating a structure of a chemical substance and an evaluation function for evaluating specific performance of the chemical substance; extracting a known chemical substance having the same basic structure as a basic structure of an input structure indicated by the input structure data from a database in which structure data indicating a structure of a chemical substance is recorded for each of a plurality of known chemical substances; generating a novel structure in which the input structure is modified based on the structure of the extracted known chemical substance or a novel structure in which the structure of the extracted known chemical substance is modified; deriving an index value related to the specific performance for the generated novel structure; deriving an evaluation value of the novel structure based on the derived index value and the evaluation function; and displaying the novel structure according to the evaluation value.

According to the disclosed technology, it is possible to support a structural design of a chemical substance exhibiting desired performance.

### BRIEF DESCRIPTION OF THE DRAWINGS

Fig. 1 is a diagram showing an example of a hardware configuration of an information processing apparatus according to an embodiment of the disclosed technology.
Fig. 2 is a diagram showing an example of structure data of a chemical substance represented in a graph format.
Fig. 3 is a diagram showing an example of a chemical substance database according to the embodiment of the disclosed technology.
Fig. 4 is a functional block diagram showing an example of a functional configuration of the information processing apparatus according to the embodiment of the disclosed technology.
Fig. 5 is a diagram showing an example of an input partial structure according to the embodiment of the disclosed technology.
Fig. 6 is a diagram showing an example of an extracted chemical structure according to the embodiment of the disclosed technology.
Fig. 7 is a diagram showing an example of a novel structure according to the embodiment of the disclosed technology.
Fig. 8 is a diagram showing an example of a display form of the novel structure according to the embodiment of the disclosed technology.
Fig. 9 is a diagram showing an example of the display form of the novel structure according to the embodiment of the disclosed technology.
Fig. 10 is a flowchart showing an example of a flow of display processing according to the embodiment of the disclosed technology.
Fig. 11 is a functional block diagram showing an example of a functional configuration of an information processing apparatus according to another embodiment of the disclosed technology.
Fig. 12 is a diagram showing an example of a partial structure database according to the embodiment of the disclosed technology.

### DESCRIPTION OF THE PREFERRED EMBODIMENTS

Hereinafter, examples of embodiments of the disclosed technology will be described with reference to the drawings. In each drawing, the same or equivalent components and parts are designated by the same references, and redundant descriptions will not be repeated as appropriate.

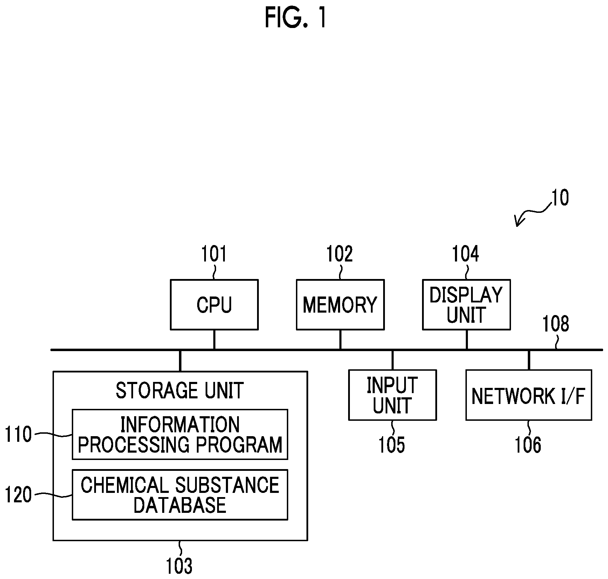

Fig. 1 is a diagram showing an example of a hardware configuration of an information processing apparatus 10 according to an embodiment of the disclosed technology.

The information processing apparatus 10 includes a central processing unit (CPU) 101, a memory 102 as a temporary storage area, and a storage unit 103. In addition, the information processing apparatus 10 includes a display unit 104 such as a liquid crystal display, an input unit 105 including an input device such as a keyboard and a mouse, and a network interface (I/F) 106 connected to a network. The CPU 101, the memory 102, the storage unit 103, the display unit 104, the input unit 105, and the network I/F 106 are each connected to a bus 108.

The storage unit 103 is realized by, for example, a nonvolatile storage medium such as a hard disk drive (HDD), a solid state drive (SSD), or a flash memory. An information processing program 110 and a chemical substance database 120 are stored in the storage unit 103. The CPU 101 reads out the information processing program 110 from the storage unit 103, then loads the information processing program 110 into the memory 102, and executes the information processing program. An example of the information processing apparatus 10 is a server computer or the like. The CPU 101 is an example of a processor in the disclosed technology.

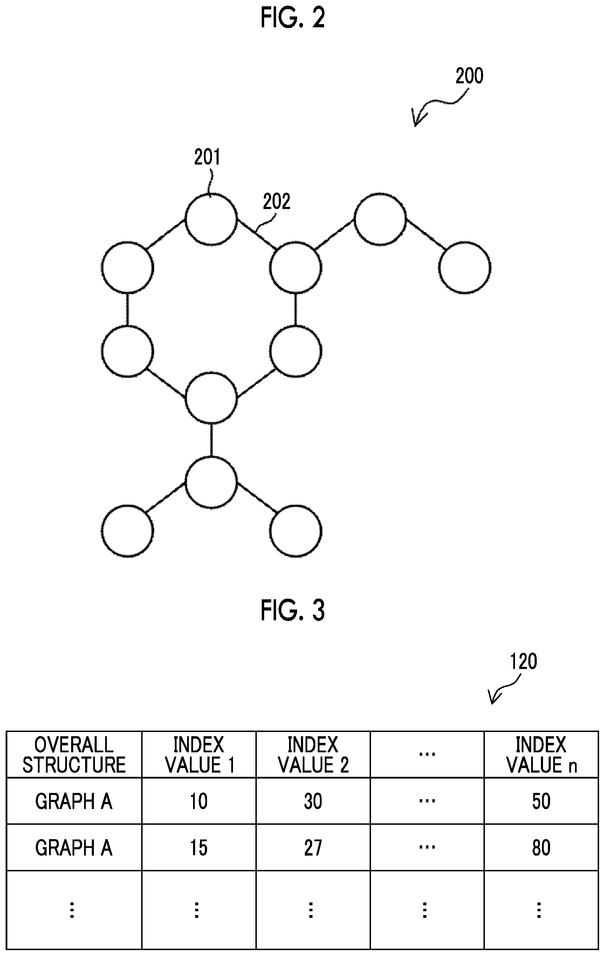

The information processing apparatus 10 is used for a structural design of a chemical substance and has a function as a molecular design editor. Structure data representing a structure of a chemical substance handled by the information processing apparatus 10 according to the present embodiment is represented in a graph format. Fig. 2 is a diagram showing an example of structure data 200 of a chemical substance represented in a graph format. In the structure data 200 represented in a graph format, atoms constituting the chemical substance are represented by nodes 201, and bonds between the atoms are represented by edges 202. The format of the structure data handled by the information processing apparatus 10 is not limited to the graph format and may be, for example, a character string format such as a deoxyribonucleic acid (DNA) base sequence.

Fig. 3 is a diagram showing an example of the chemical substance database 120 stored in the storage unit 103. The chemical substance database 120 has recorded therein structure data representing an overall structure of the chemical substance for each of a plurality of known chemical substances. The structure data is represented in a graph format. At least one index value representing the performance of the chemical substance is associated with each piece of the structure data. Examples of the index value include a boiling point, a melting point, a glass transition temperature, a partition coefficient, a density, a viscosity, a thermal expansion factor, and a molecular weight. The index value may be, for example, an actually measured value obtained by a past experiment or a nominal value.

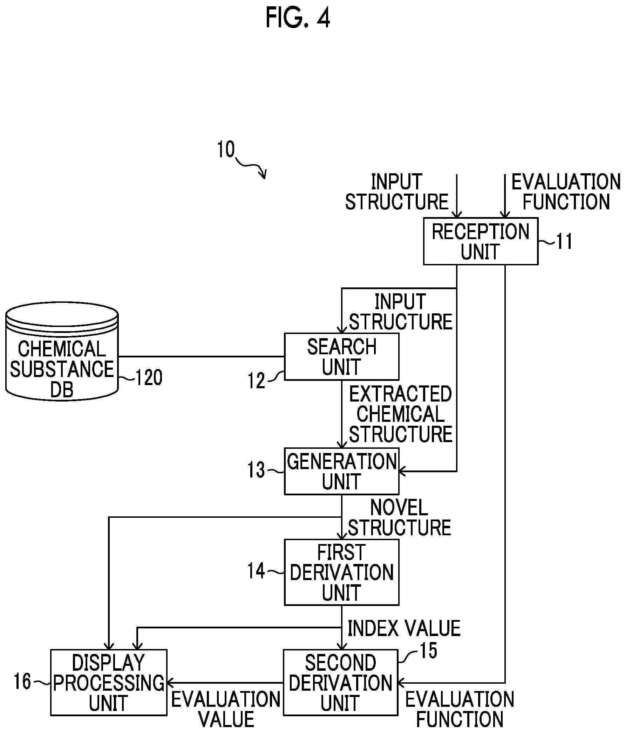

Fig. 4 is a functional block diagram showing an example of a functional configuration of the information processing apparatus 10. The information processing apparatus 10 includes a reception unit 11, a search unit 12, a generation unit 13, a first derivation unit 14, a second derivation unit 15, and a display processing unit 16. By executing the information processing program 110 by the CPU 101, the information processing apparatus 10 functions as the reception unit 11, the search unit 12, the generation unit 13, the first derivation unit 14, the second derivation unit 15, and the display processing unit 16.

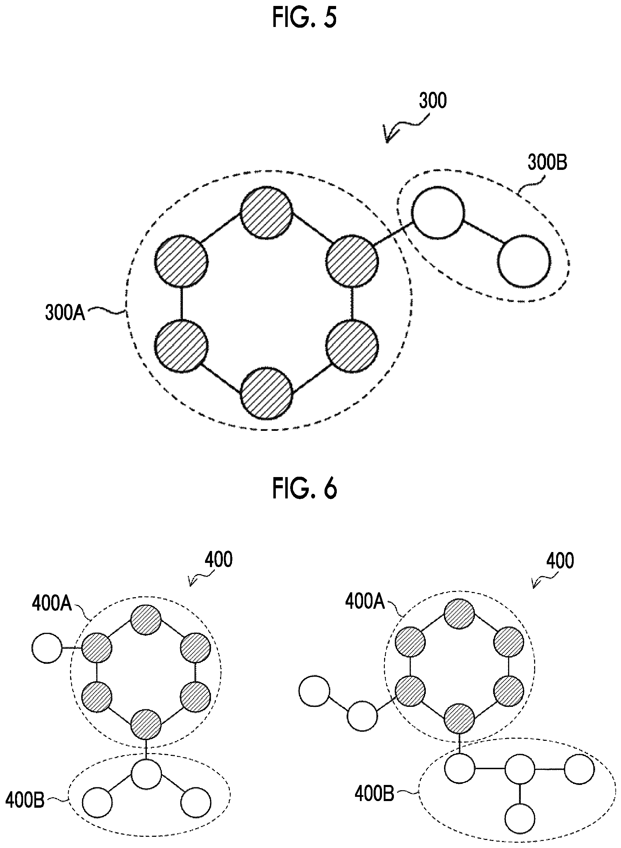

A user who performs a structural design of a chemical substance using the information processing apparatus 10 inputs to the information processing apparatus 10 a chemical structure that can be included in the chemical substance to be designed. The chemical structure input to the information processing apparatus 10 is hereinafter referred to as an input structure. Fig. 5 is a diagram showing an example of an input structure 300. In Fig. 5, in the input structure 300, nodes forming a basic structure 300A are hatched. The basic structure will be described later. The input structure can be input to the information processing apparatus 10 by operating the input unit 105. The reception unit 11 receives structure data indicating the input structure input by the user and supplies the structure data to the search unit 12 and the generation unit 13.

In addition, the user inputs an evaluation function for evaluating specific performance of the chemical substance to the information processing apparatus 10. An evaluation value evaluating performance of a novel structure generated by the generation unit 13 is derived using the evaluation function. The evaluation function is formulated such that the closer the performance of the generated novel structure is to a target, the higher the evaluation value is. For example, in a case where target values are set for the boiling point and the partition coefficient, and the structure of the chemical substance is designed, the boiling point and the partition coefficient of the novel structure are used as variables of the evaluation function. The evaluation function is formulated such that the closer the boiling point and the partition coefficient of the novel structure are to the target, the higher the evaluation value is. The details of the novel structure will be described later. The evaluation function can be input to the information processing apparatus 10 by operating the input unit 105. The reception unit 11 receives the evaluation function input by the user and supplies the evaluation function to the second derivation unit 15.

The search unit 12 searches for and extracts from the chemical substance database 120 a known chemical substance that has the same basic structure as a basic structure of the input structure received by the reception unit 11. The basic structure is a structure forming a skeleton of a chemical substance, and may be, for example, a structure corresponding to a main chain. The basic structure may be a predefined structure. In a case where a plurality of known chemical substances having the same basic structure as the basic structure of the input structure are present in the chemical substance database 120, the search unit 12 extracts all the corresponding chemical substances. Hereinafter, the structure of the chemical substance extracted by the search unit 12 will be referred to as an extracted chemical structure. Fig. 6 is a diagram showing an example of an extracted chemical structure 400. In Fig. 6, in the extracted chemical structure 400, nodes forming a basic structure 400A are hatched. The basic structure 400A in two extracted chemical structures 400 shown in Fig. 6 is the same as the basic structure 300A in the input structure 300 shown in Fig. 5. The search unit 12 supplies structure data indicating the extracted chemical structure to the generation unit 13.

The generation unit 13 generates a novel structure in which the input structure is modified based on the extracted chemical structure. For example, the generation unit 13 generates a novel structure by adding a partial structure associated with the basic structure of the extracted chemical structure to the input structure. In addition, the generation unit 13 generates a novel structure by deleting the partial structure associated with the basic structure of the input structure from the input structure. The partial structure is a part of a structure constituting a chemical substance, and is a structure associated with the basic structure.

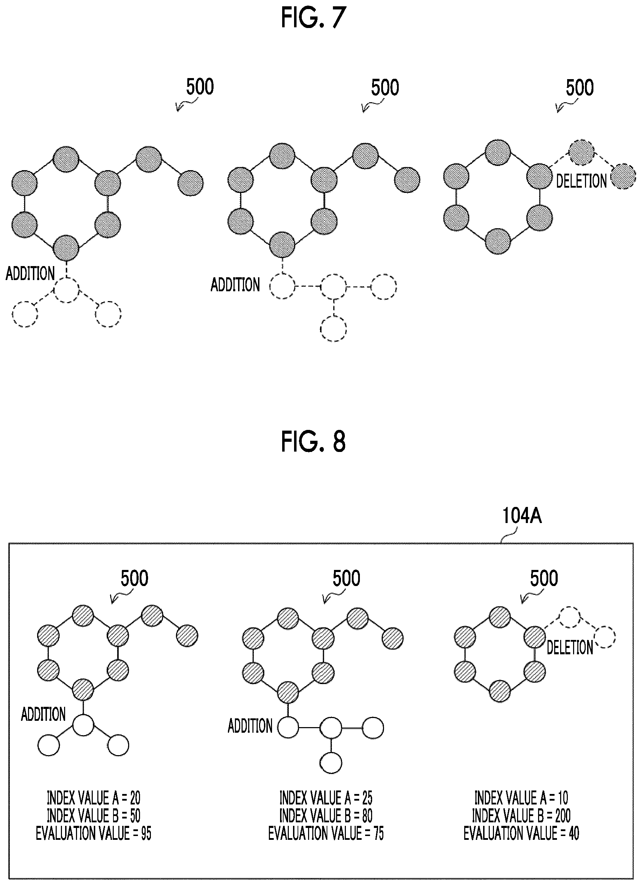

Fig. 7 is a diagram showing an example of a novel structure 500 generated by the generation unit 13. The novel structure 500 shown on the left side of Fig. 7 is obtained by adding a partial structure 400B associated with the lowermost part of the basic structure 400A of the extracted chemical structure 400 shown on the left side of Fig. 6 to a corresponding portion of the input structure 300 shown in Fig. 5. On the left side of Fig. 7, a structure corresponding to the input structure is shown by hatching, and the partial structure added to the input structure is shown by a broken line. The novel structure 500 shown in the center of Fig. 7 is obtained by adding the partial structure 400B associated with the lowermost part of the basic structure 400A of the extracted chemical structure 400 shown on the right side of Fig.

6 to a corresponding portion of the input structure 300 shown in Fig. 5. In the center of Fig. 7, a structure corresponding to the input structure is shown by hatching, and the partial structure added to the input structure is shown by a broken line. The novel structure 500 shown on the right side of Fig. 7 is obtained by deleting the partial structure 300B associated with the basic structure 300A of the input structure 300 shown in Fig. 5 from the input structure 300. On the right side of Fig. 7, a structure corresponding to the input structure is shown by hatching, and the partial structure deleted from the input structure is shown by a broken line.

The generation unit 13 generates a novel structure such that the novel structure is different from a structure of a known chemical substance recorded in the chemical substance database 120. The generation unit 13 supplies the generated novel structure to the first derivation unit 14 and the display processing unit 16.

The first derivation unit 14 derives an index value related to the performance of the novel structure generated by the generation unit 13. The index value derived by the first derivation unit 14 includes a value related to the performance set as a variable in the evaluation function received by the reception unit 11. For example, in a case where the boiling point and the partition coefficient are set as variables of the evaluation function, the first derivation unit 14 derives at least the boiling point and the partition coefficient for the novel structure. The first derivation unit 14 may derive an index value by using, for example, a known estimation method such as a quantitative structure-activity relationship (QSAR). The QSAR is a method of estimating physical properties of a chemical substance based on a chemical structure using a mathematical model. In a case where a plurality of novel structures are generated by the generation unit 13, the first derivation unit 14 derives an index value for each of the plurality of novel structures. The first derivation unit 14 supplies the derived index value to the second derivation unit 15 and the display processing unit 16.

The second derivation unit 15 derives the evaluation value for the novel structure by substituting the index value derived by the first derivation unit 14 for the variable of the evaluation function. The evaluation value is a numerical value that evaluates a specific performance of the novel structure. The higher the evaluation value derived by the second derivation unit 15, the closer the performance of the novel structure is to the target. In a case where a plurality of novel structures are generated by the generation unit 13, the second derivation unit 15 derives an evaluation value for each of the plurality of novel structures. The second derivation unit 15 supplies the derived evaluation value to the display processing unit 16.

The display processing unit 16 performs a process of displaying the novel structure generated by the generation unit 13 on the display unit 104 according to the evaluation value derived by the second derivation unit 15. Fig. 8 is a diagram showing an example of a display form of the novel structure 500 displayed on a display screen 104A of the display unit 104. The display processing unit 16 performs a process of displaying a difference between the novel structure 500 and the input structure in a recognizable manner. For example, in the novel structure 500, the partial structure added to the input structure may be displayed in a color different from that of the input structure. In addition, the partial structure deleted from the input structure may be displayed in a blinking manner.

In a case where a plurality of novel structures are generated, the display processing unit 16 ranks the plurality of novel structures based on the evaluation value and displays the plurality of novel structures 500 in a manner in which ranking results are recognizable. For example, as illustrated in Fig. 8, a process of displaying the plurality of novel structures 500 in order from the left to the right of the display screen 104A in descending order of the evaluation value is performed. The plurality of novel structures may be displayed in order from the top to the bottom of the display screen 104A in descending order of the evaluation value. In addition, the display processing unit 16 performs a process of displaying the index value and the evaluation value derived for the novel structure 500 together with the novel structure 500. As the index value, only those related to the performance set as variables in the evaluation function (that is, those contributing to the evaluation value) may be selectively displayed.

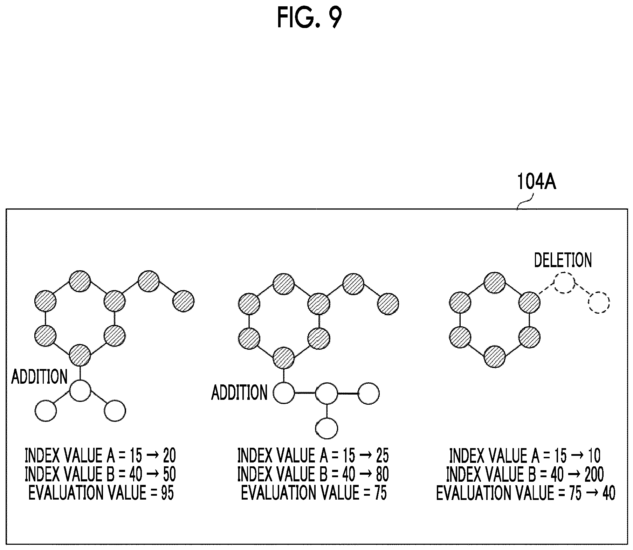

In addition, as illustrated in Fig. 9, the display processing unit 16 may perform a process of explicitly displaying how the index value related to the specific performance of the novel structure has changed with respect to the input structure. Fig. 9 illustrates an example of a display form in which both the index value in the input structure and the index value in the novel structure are displayed. In this case, the first derivation unit 14 derives the index value not only for the novel structure but also for the input structure. In addition, in a case where a plurality of novel structures are generated, the display processing unit 16 may perform a process of displaying only the novel structures of which the evaluation values are equal to or greater than a threshold value among the plurality of novel structures.

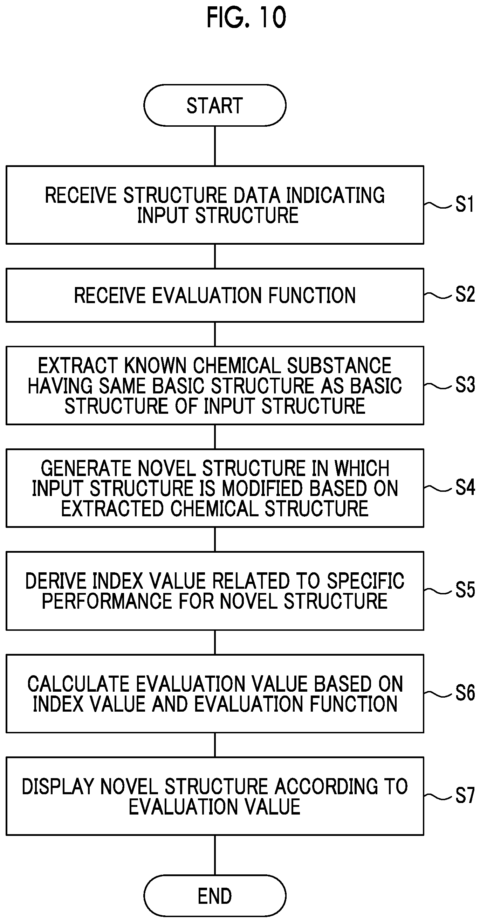

Fig. 10 is a flowchart showing an example of a flow of display processing implemented by executing the information processing program 110 by the CPU 101. In step S 1, the reception unit 11 receives structure data indicating the input structure input by the user by operating the input unit 105. In step S2, the reception unit 11 receives the evaluation function input by the user by operating the input unit 105.

In step S3, the search unit 12 searches for and extracts from the chemical substance database 120 a known chemical structure that has the same basic structure as the basic structure of the input structure received in step S1.

In step S4, the generation unit 13 generates a novel structure in which the input structure received in step S1 is modified based on the structure (that is, the extracted chemical structure) of the known chemical substance extracted in step S3. The generation unit 13 generates a novel structure, for example, by adding a partial structure associated with the basic structure of the extracted known chemical substance to the input structure. In addition, the generation unit 13 generates a novel structure, for example, by deleting a partial structure associated with the basic structure of the input structure from the input structure.

In step S5, the first derivation unit 14 derives an index value related to specific performance for the novel structure generated in step S4. The index values derived in this step include those related to the performance set as variables in the evaluation function.

In step S6, the second derivation unit 15 derives the evaluation value for the novel structure based on the index value derived in Step S5 and the evaluation function received in step S2.

In step S7, the display processing unit 16 performs a process of displaying the novel structure generated in step S4 on the display unit 104 in accordance with the evaluation value derived in step S6. For example, in a case where a plurality of novel structures are generated, the display processing unit 16 ranks the plurality of novel structures based on the evaluation values and displays the plurality of novel structures in a manner in which the ranking result is recognizable.

As described above, the information processing apparatus 10 according to the embodiment of the disclosed technology generates a novel structure in which the input structure is modified based on the structure of a known chemical substance having the same basic structure as a basic structure of the input structure, and displays the novel structure according to the evaluation value derived for the novel structure. According to the information processing apparatus 10, since the novel structure is presented to the user in a display mode based on the evaluation value, it is possible to support the structural design of the chemical substance exhibiting desired performance.

In addition, since the novel structure is generated based on a known chemical structure having the same basic structure as the basic structure of the input structure, it is possible to generate a novel structure with high feasibility as compared with a case where a novel structure is randomly generated. In addition, by displaying a difference between the novel structure and the input structure in a recognizable manner, it becomes easy to understand the partial structure added to or deleted from the input structure. In addition, by displaying a plurality of novel structures in a manner in which a result of ranking according to the evaluation value is recognizable, it becomes easy to understand a novel structure having the most desirable performance from among the plurality of novel structures. In addition, as shown in Fig. 10, by displaying the index values derived for each of the input structure and the novel structure, it is possible to understand how the index value in the novel structure has changed with respect to the input structure. In addition, by displaying only the novel structure of which the evaluation value is equal to or greater than the threshold value among the plurality of novel structures, it is possible to present only the novel structure having desirable performance to the user.

In addition, in the above-described embodiment, a case where the generation unit 13 generates a novel structure by modifying the input structure based on a known chemical structure (that is, an extracted chemical structure) having the same basic structure as the basic structure of the input structure is illustrated. However, the disclosed technology is not limited to this aspect. The generation unit 13 may generate a novel structure by modifying the extracted chemical structure. For example, a novel structure may be generated by changing a connection position of the partial structure 400B associated with the basic structure 400A of the extracted chemical structure 400. In addition, a novel structure may be generated by adding the partial structure 400B of another extracted chemical structure 400 to the basic structure 400A of the extracted chemical structure 400. In addition, a novel structure may be generated by replacing the partial structure 400B of the extracted chemical structure 400 with another partial structure 400B of the extracted chemical structure 400. In addition, a novel structure may be generated by deleting the partial structure 400B of the extracted chemical structure 400. In addition, a novel structure may be generated by a combination of addition, replacement, or deletion of the partial structure described above.

### [Second Embodiment]

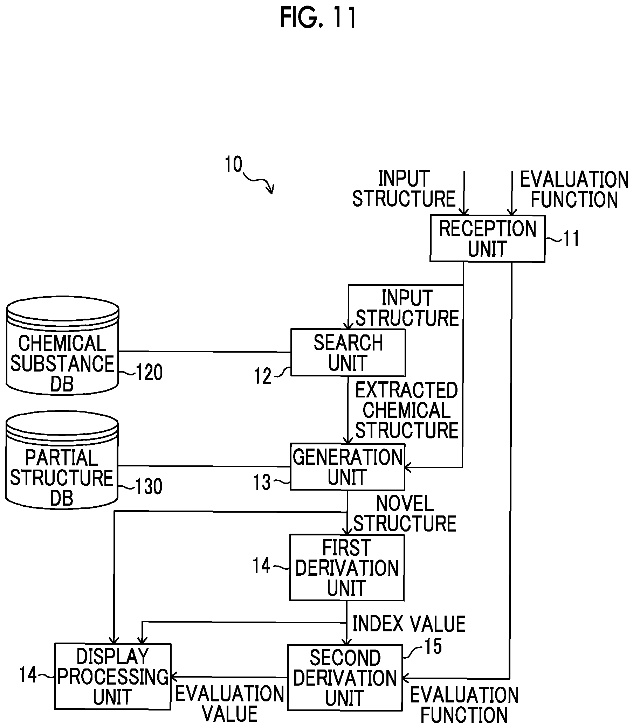

Fig. 11 is a functional block diagram showing an example of a functional configuration of the information processing apparatus 10 according to a second embodiment of the disclosed technology. The information processing apparatus 10 according to the second embodiment includes a partial structure database 130. The partial structure database 130 is stored in the storage unit 103.

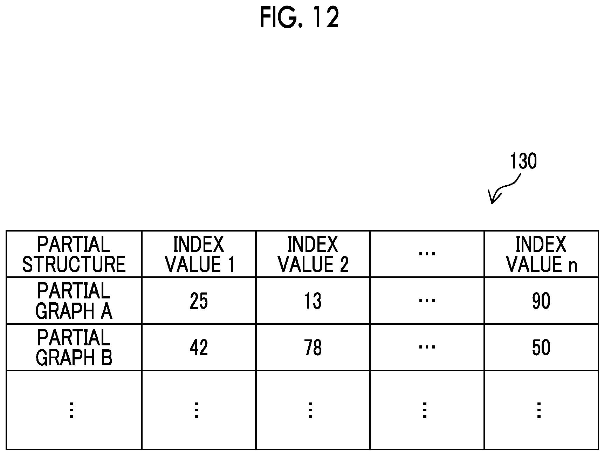

Fig. 12 is a diagram showing an example of the partial structure database 130. The partial structure database 130 has recorded therein partial structure data representing the partial structure for each of a plurality of known partial structures. In the partial structure database, for example, a structure of a functional group such as a carboxyl group, an aldehyde group, or a hydroxyl group is recorded as a partial structure. The structure data of the partial structure is represented in a graph format. At least one index value representing performance of the partial structure is associated with each piece of the structure data of the partial structure. Examples of the index value include presence or absence of carcinogenicity, presence or absence of toxicity, and a degree indicating a solubility in water. The index value may be, for example, an actually measured value obtained by a past experiment or a nominal value.

As in the first embodiment, the generation unit 13 generates a novel structure in which the input structure is modified based on the extracted chemical structure illustrated in Fig. 6. For example, the generation unit 13 generates a novel structure by adding a partial structure associated with the basic structure of the extracted chemical structure to the input structure. In addition, the generation unit 13 generates a novel structure by deleting the partial structure associated with the basic structure of the input structure from the input structure.

In the present embodiment, the generation unit 13 determines a partial structure to be added to the input structure and a partial structure to be deleted from the input structure by referring to the partial structure database 130. In a case where the generation unit 13 finds the same partial structure as the partial structure recorded in the partial structure database 130 in the extracted chemical structure illustrated in Fig. 6, the generation unit 13 determines whether or not performance of the partial structure satisfies a predetermined condition. This determination is made based on the index value recorded corresponding to the partial structure in the partial structure database 130. The conditions are set in advance by the user. The conditions include, for example, that a toxicity level of the partial structure is equal to or less than a threshold value. In a case where the generation unit 13 determines that the performance of the partial structure satisfies the condition, the generation unit 13 targets the partial structure to be added to the input structure. On the other hand, in a case where the generation unit 13 determines that the performance of the partial structure does not satisfy the condition, the generation unit 13 excludes the partial structure from the target to be added to the input structure. Thereby, it is possible to suppress addition of the partial structure having undesirable performance to the input structure.

In addition, in a case where the generation unit 13 finds the same partial structure as the partial structure recorded in the partial structure database 130 in the input structure illustrated in Fig. 5, the generation unit 13 determines whether or not the performance of the partial structure satisfies a predetermined condition. This determination is made based on the index value recorded corresponding to the partial structure in the partial structure database 130. The conditions are set in advance by the user. The conditions include, for example, that specific performance of the partial structure satisfies requirements. In a case where the generation unit 13 determines that the performance of the partial structure does not satisfy the condition, the generation unit 13 targets the partial structure to be deleted from the input structure. On the other hand, in a case where the generation unit 13 determines that the performance of the partial structure satisfies the condition, the generation unit 13 excludes the partial structure from the target to be deleted from the input structure. Thereby, it is possible to suppress deletion of the partial structure having desirable performance from the input structure.

In this way, by referring to the partial structure database 130 and selecting the partial structure to be added to the input structure and the partial structure to be deleted from the input structure, it is possible to suppress generation of a novel structure that does not meet performance requirements.

In the above-described embodiment, for example, as a hardware structure of a processing unit that executes various types of processing such as the reception unit 11, the search unit 12, the generation unit 13, the first derivation unit 14, the second derivation unit 15, and the display processing unit 16, various types of processors shown below can be used. As described above, in addition to the CPU which is a general-purpose processor executing software (program) to function as various types of processing units, the various types of processors include a programmable logic device (PLD) which is a processor capable of changing a circuit configuration after manufacture such as a field programmable gate array (FPGA), a dedicated electric circuitry which is a processor having a circuit configuration exclusively designed to execute specific processing such as an application specific integrated circuit (ASIC), and the like.

One processing unit may be configured of one of the various types of processors, or a combination of two or more processors of the same type or different types (for example, a combination of a plurality of FPGAs, or a combination of a CPU and an FPGA). In addition, a plurality of processing units may be configured by one processor.

As an example of configuring a plurality of processing units with one processor, first, there is a form in which, as typified by computers such as a client and a server, one processor is configured by combining one or more CPUs and software, and the processor functions as a plurality of processing units. Second, as typified by a system on chip (SoC) or the like, there is a form in which a processor that realizes functions of an entire system including a plurality of processing units with one integrated circuit (IC) chip is used. As described above, the various types of processing units are configured using one or more of the various types of processors as a hardware structure.

Furthermore, as the hardware structure of the various types of processors, more specifically, an electric circuitry in which circuit elements such as semiconductor elements are combined can be used.

Further, in the above-described embodiment, an aspect in which the information processing program 110 is stored (installed) in advance in the storage unit 103, but the disclosed technology is not limited thereto. The information processing program 110 may be provided in a form recorded in a recording medium such as a compact disc read only memory (CD-ROM), a digital versatile disc read only memory (DVD-ROM), and a universal serial bus (USB) memory. Further, the information processing program 110 may be downloaded from an external device via a network.

The disclosure of JP 2021-001611 filed on January 7, 2021 is incorporated herein by reference in its entirety. In addition, all publications, patent applications, and technical standards described in this specification are incorporated by reference herein to the same extent as in a case where it is specifically and individually stated that individual documents, patent applications, and technical standards are incorporated by reference.

## Claims

1. An information processing apparatus comprising at least one processor,
wherein the processor is configured to:
receive input of structure data indicating a structure of a chemical substance and an evaluation function for evaluating specific performance of the chemical substance;
extract a known chemical substance having the same basic structure as a basic structure of an input structure indicated by the input structure data from a database in which structure data indicating a structure of a chemical substance is recorded for each of a plurality of known chemical substances;
generate a novel structure in which the input structure is modified based on the structure of the extracted known chemical substance or a novel structure in which the structure of the extracted known chemical substance is modified;
derive an index value related to the specific performance for the generated novel structure;
derive an evaluation value of the novel structure based on the derived index value and the evaluation function; and
display the novel structure according to the evaluation value.

2. The information processing apparatus according to claim 1, wherein the processor is configured to generate the novel structure by adding a partial structure associated with the basic structure of the extracted known chemical substance to the input structure.

3. The information processing apparatus according to claim 2, wherein the processor is configured to generate the novel structure by deleting a partial structure associated with the basic structure of the input structure from the input structure.

4. The information processing apparatus according to any one of claims 1 to 3, wherein the processor is configured to display a difference between the novel structure and the input structure in a recognizable manner.

5. The information processing apparatus according to any one of claims 1 to 4, wherein the processor is configured to:
rank a plurality of the novel structures based on the evaluation value; and
display the plurality of the novel structures in a manner in which a result of the ranking is recognizable.

6. The information processing apparatus according to any one of claims 1 to 5, wherein the processor is configured to:
derive an index value related to the specific performance for the input structure; and
display the index values derived for each of the input structure and the novel structure.

7. The information processing apparatus according to any one of claims 1 to 6, wherein the processor is configured to display only a novel structure of which the evaluation value is equal to or greater than a threshold value among a plurality of the generated novel structures.

8. An information processing method in which a processor of an information processing apparatus executes a process comprising:
receiving input of structure data indicating a structure of a chemical substance and an evaluation function for evaluating specific performance of the chemical substance;
extracting a known chemical substance having the same basic structure as a basic structure of an input structure indicated by the input structure data from a database in which structure data indicating a structure of a chemical substance is recorded for each of a plurality of known chemical substances;
generating a novel structure in which the input structure is modified based on the structure of the extracted known chemical substance or a novel structure in which the structure of the extracted known chemical substance is modified;
deriving an index value related to the specific performance for the generated novel structure;
deriving an evaluation value of the novel structure based on the derived index value and the evaluation function; and
displaying the novel structure according to the evaluation value.

9. An information processing program for causing a processor of an information processing apparatus to execute a process comprising:
receiving input of structure data indicating a structure of a chemical substance and an evaluation function for evaluating specific performance of the chemical substance;
extracting a known chemical substance having the same basic structure as a basic structure of an input structure indicated by the input structure data from a database in which structure data indicating a structure of a chemical substance is recorded for each of a plurality of known chemical substances;
generating a novel structure in which the input structure is modified based on the structure of the extracted known chemical substance or a novel structure in which the structure of the extracted known chemical substance is modified;
deriving an index value related to the specific performance for the generated novel structure;
deriving an evaluation value of the novel structure based on the derived index value and the evaluation function; and
displaying the novel structure according to the evaluation value.
